# EUROPEAN PATENT APPLICATION

(11) **EP 3 534 156 A1**
(43) Date of publication of application: **04.09.2019**
(21) Application number: 17864999.2
(22) Date of filing: 26.10.2017
(51) Int. Cl.: G01N 31/22, G01N 33/18, C08L 33/26

(54) **PORTABLE MICROFLUIDIC DEVICE FOR DETECTING NITRITE-NITRATE**

(30) Priority: 26.10.2016 ES 201631376
(71) Applicant: Universidad del Pais Vasco - Euskal Herriko Unibertsitatea (UPV/EHU), 48940 Leioa (ES)
(72) Inventor: BENITO LÓPEZ, Fernando, 48940 Leioa - Vizcaya (ES); SAEZ CASTAÑO, Janire, 48940 Leioa - Vizcaya (ES)
(74) Representative: Balder IP Law, S.L.
(86) International application number: PCT/ES2017/070719
(87) International publication number: WO 2018/078208

(57) **Abstract**

The invention relates to an ion gel comprising a hydrogel in an ionic liquid, and a reactive system that changes colour upon reaction with the nitrite. The invention also relates to a microfluidic device comprising a substrate (1) having calibration reservoirs (2) that are separated from one another and include an ion gel, a calibration reservoir (3) of the hydrogel in the ionic liquid, a reservoir (6) for holding a sample to be analysed, connected to a measuring reservoir (4) including an ion gel via a microfluidic channel (5) with a reducing agent, and a measuring reservoir (7) including an ion gel of the invention where a sample to be analysed is held. The invention further relates to methods for producing the ion gel and the device, as well as a method for colorimetrically determining and detecting nitrite and nitrate in contaminated water using the ion gel and the device.

## Description

### Field of the invention

The present invention is comprised among techniques for detecting and determining the nitrate contamination of a mass of water. It particularly relates to a portable microfluidic device capable of detecting combined nitrite-nitrate in contaminated water in a quick and effective manner, and at the actual site of the contamination.

### Background of the invention

In recent years, various environmental monitoring devices have emerged in response to the growing contamination of natural, industrial and municipal water with toxic substances contaminating the water, such as harmful chemical agents. Environmental monitoring agencies generally work by hand, by means of taking samples at the contaminated site, and transporting them to the corresponding facilities where the analysis is carried out by highly qualified personnel using sophisticated instruments. This strategy has benefits such as obtaining very precise and accurate results and compliance with legal regulating proceedings, but it has drawbacks such as the high costs associated with maintaining these facilities, the instruments and the qualified personnel. Furthermore, protocols of this type impede working *in situ,* thereby prolonging response times in the event of an unexpected contamination. Other types of methods allow obtaining information *in situ,* but the equipment used is generally expensive and sophisticated, so it also requires qualified personnel for use.

The determination of the concentration of nutrients in a mass of water is very important because the concentration of nutrients affects and modifies the equilibrium of life in the water. The uncontrolled increase in the concentration of nutrients in the water could lead to an environmental disaster. In particular, the increase in nitrates is detrimental to human health and provokes degradation of aquatic life, such as eutrophication of algea, which leads to the death of fauna and flora.

Current techniques for detecting nitrate and nitrite are amperometric, electrochemical, biosensing, or based on spectrophotometric methods, which are the most popular methods due to the excellent limits of detection (nanomolar), dynamic range and the cost efficiency.

Classic colorimetric methods have conventionally been applied as a rapid tool for determining and detecting the concentration of analytes in several matrices.

The colorimetric technique uses the capacity of the reagents to bind to analytes of interest which generate a coloured reaction. The intensity of this reaction will directly depend on the concentration of the analyte. In particular, the Griess reagent is known and used for determining and detecting nitrite in an aqueous matrix. Colorimetrically determining nitrite by means of the Griess reagent is chemically robust, offers excellent analytical performance and has been applied in the development of several analytical platforms. However, these systems are generally complicated devices consisting of elements for holding reagents, pumps and valves for the control of the flow of liquids in the device and detection modules, rendering them expensive and unsuitable for use at the site of the contamination.

The present invention has arisen in view of the need in the state of the art to provide systems for detecting combined nitrite/nitrate, alternatives that overcome at least some of the mentioned drawbacks of the methods today.

### Description of Figures

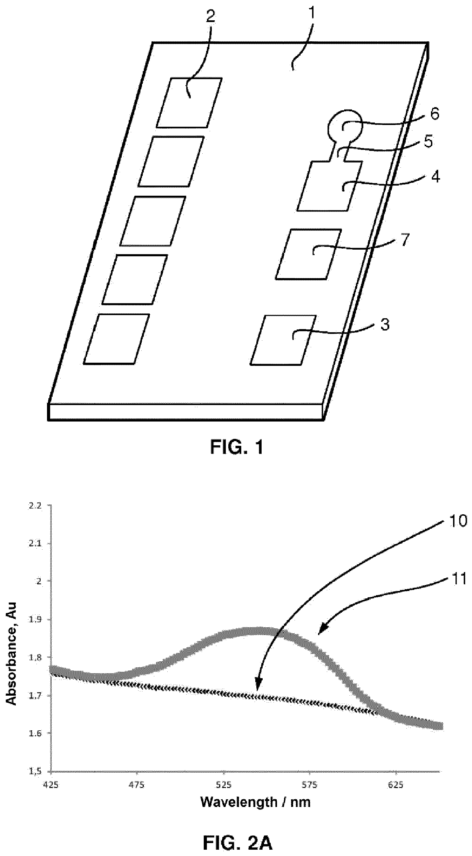

**Figure 1****:** depiction of a microfluidic device of the invention consisting of a substrate (1), five calibration reservoirs (2), a reservoir (6) for holding a sample to be analysed, a calibration reservoir (3) for calibrating the "target", a measuring reservoir (4), a measuring reservoir (7) for holding a sample to be analysed, and a microfluidic channel (5) communicating reservoir (6) and reservoir (4), and including a reducing agent.
**Figure 2A****:** shows a UV/UV-Vis spectrum of ion gel 1 (IO-1) comprising a hydrogel in an ionic liquid with Griess reagent (10) and of said hydrogel in the ionic liquid with Griess reagent and with nitrite (11).

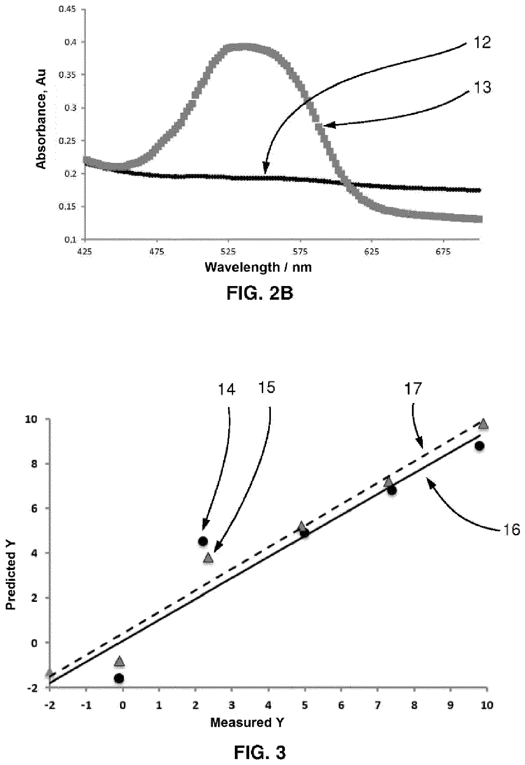

**Figure 2B****:** shows the UV/UV-Vis spectrum of another ion gel 2 (IO-2) comprising another hydrogel in another ionic liquid with Griess reagent (12) and said hydrogel in said ionic liquid with Griess reagent and with nitrite (13). The maximum absorbance for the Griess reaction with nitrite was found at λ = 532 nm. The differences in absorbance are observed in these Figures 2A and 2B.
**Figure 3****:** represents the curves of a multivariate calibration (17) formed by triangles (15), and validation (16) model, formed by dots (14), generated after analysis by imaging of the concentrations: (0 ppm), (2.5 ppm), (5 ppm), (7.5 ppm) and (10 ppm).

### Description of the invention

In one aspect, the invention relates to an ion gel, hereinafter ion gel of the invention, comprising:
- a hydrogel in an ionic liquid, and
- a reactive system providing a change in colour upon reaction with the nitrite.

The hydrogel is a polymerised and cross-linked porous structure with hydrophilic properties capable of retaining a considerable fraction of water in the same. While the hydrogels are generally prepared from hydrophilic monomers, hydrophobic monomers can also be used to regulate properties for specific applications.

According to the present invention, the hydrogel is obtained in an ionic liquid by the polymerisation and cross-linking of two or more precursor monomers selected from acrylic acid, alkali metal acrylates, acrylamide and acrylic derivatives thereof, well known to one skilled in the art, such as methyl acrylate, methyl methacrylate, etc. In a particular embodiment, the hydrogel is a polyacrylamide.

In a preferred embodiment, the hydrogel is obtained by polymerisation of N-isopropylacrylamide and N,N'-methylenebis(acrylamide) which, in addition to being a co-monomer, is a cross-linking agent. The ratios of the monomers in the final hydrogel polymer range between ample margins and are easily determined by a skilled person depending on the desired hardness and degree of cross-linking. For example, in the case of N-isopropylacrylamide and N,N'-methylene-bis(acrylamide), the ratios can range from 100:1 to 10:1, and for example can be 90:1, 70:1, 50:1 or 20:1.

The reactive system providing a change in colour upon reaction with the nitrite which can be used in the present invention can theoretically be any conventional colorimetric reactive system known to one skilled in the art. Non-limiting examples of said reactive systems are enzymes such as nitrite and nitrate reductases, which are described for example in "Nitrite Biosensing via Selective Enzymes-A Long but Promising Route; M. Gabriela Almeida, Alexandra Serra, Celia M. Silveira, and Jose J.G. Moura; Sensors (Basel). 2010; 10(12): 11530-11555", diphenylbenzidine, the minoxidil described in "Detection of Nitrite in Water Using Minoxidil as a Reagent, Mario Gonzalez-Jimenez, Jorge Arenas-25 Valganon, Isaac F. Cespedes-Camacho, Juan Carlos Garefa-Prieto, Emillio Calle, and Julio Casado, J.Chem.Educ; 2013, 90, 1053-1056", or the Griess reagent. In a preferred embodiment of the invention, the Griess reagent is used.

The ionic liquids are organic salts that are liquid at temperatures close to room temperature and comprise an organic cation and an anion which can be organic or inorganic. They have notable properties such as zero volatility, high ionic conductivity, as well as catalytic properties. They are used today in a number of fields, in particular as electrolytes. Among the possible organic cations that can be used in the present invention there are included imidazolium, pyridinium, pyrrolidinium, ammonium, or phosphonium derivatives. Among others, 1-alkyl-3-methylimidazolium, 1-alkylpyridinium, N-methyl-N-alkylpyrrolidinium, ammonium salts, phosphonium salts, etc., stand out. Non-limiting examples of cations are trihexyltetradecyl phosphonium [P6,6,6,14]⁺; tributyl tetradecyl phosphonium [P4,4,4,14]⁺; tretrabutyl phosphonium [P4,4,4]⁺; triisobutyl methyl phosphonium [P1,4,4,4]⁺; 1-butyl-1-methyl pyrrolidine, 1-ethyl-3-methylimidazolium [emim]⁺, 1-butyl-3-methylimidazolium [bmim]⁺, N-propyl N-methyl-pyrrolidinium [C₃mpyr]⁺, N-butyl N-methyl-pyrrolidinium [C₄mpyr]⁺, trioctyl methyl ammonium [Oct₃NMe]⁺.

Among the possible anions that can be used in the present invention, there are included sulphonates, borates, phosphates, halides, etc. Non-limiting examples of anions are, among others, tosylate [tos]-dodecylbenzenesulphonate [dbsa]⁻, ethyl sulphate, bis(trifluoromethanesulphonyl) amide [NTf₂]⁻, dicyanamide [dca]⁻, tetracyanoborate [BCN₃]⁻, hexafluorophosphate [PF₆]⁻ tetrafluoroborate [BF₄]⁻, ethyl sulphate, tris(pentafluorethyl)trifluorophosphate, chloride, bromide, iodide, fluoride.

In a particular embodiment, the ionic liquid is 1-ethyl-3-methylimidazolium ethyl sulphate (IO-1). In another particular embodiment, the ionic liquid is trihexyltetradecyl phosphonium dicyanamide (IO-2).

The reactive system is embedded within the hydrogel in the ionic liquid, the structure of which allows storing the reactive system for long periods of time without it deteriorating, while at the same time it acts as a colorimetric sensor, as explained below.

In another aspect, the invention relates to a microfluidic device comprising:
a) a substrate 1 comprising:
b) two or more calibration reservoirs 2 that are separated from one another and include an ion gel of the invention,
c) a calibration reservoir 3 comprising hydrogel in the ionic liquid ("target"),
d) a reservoir 6 for holding a sample to be analysed connected to a measuring reservoir 4 including an ion gel of the invention via a microfluidic channel including a reducing agent,
e) a measuring reservoir 7 including an ion gel of the invention where a sample to be analysed is held.

The ion gel of the invention which is used in a particular microfluidic device is the same in all the reservoirs; where this ion gel comprises a given hydrogel in a given ionic liquid which is used in the calibration reservoir 3 ("target").

The substrate 1 used as a support for the device is a plastic plate. The plate can theoretically be of any material provided that it is inert to the materials with which it will be in contact, i.e., it does not react and does not interfere with the chemistry of the microfluidic device sensor and remains unchanged.

Examples of plastics suitable for the substrate are, among others, high-density polyethylene, low-density polyethylene, ethylene polyterephthalate, polyvinyl chloride, polypropylene, polystyrene or polycarbonate, cyclic olefin polymer or copolymer or acrylic resins. According to a particular embodiment, the substrate is a poly(methyl methacrylate) plate. These plates can be obtained commercially.

The dimensions of the substrate can vary and there is no particular limitation in this regard. The device size can vary depending on the analysis requirements, the number of samples to be analysed in a single device, the number of repetitions to be made of a given sample, among other parameters. Ideally the size is defined such that it can be easily handled in one hand (portable). In a particular embodiment, the device is 1 mm thick and has a size of 22 mm x 10 mm.

Likewise, the dimensions and shapes of the reservoirs can also vary in the device, without there being any particular limitation in this regard either.

The precision of the device when determining and quantifying the nitrite content in a sample will depend, among other factors, on the number of calibration reservoirs 2 that are separated from one another. In this sense, the device preferably has at least 3, preferably at least 4, and more preferably at least 5 calibration reservoirs 2. These reservoirs contain different concentrations of nitrite (standard solutions) and a change in colour is caused therein upon reaction of the reactive system with the nitrite which is proportional in colour intensity to the concentration of nitrite in a linear range.

According to a particular embodiment, the microfluidic channel 5 comprises a microfluidic paper. A reducing agent is in turn arranged in said channel, which reducing agent reduces the nitrate present in the sample to be analysed arranged in the reservoir 6 to nitrite, which is the analyte for which the reactive system of the ion gel of the invention shows sensitivity. In a particular embodiment, the reducing agent comprises Zn(0) and is arranged, for example, in the form of an emulsion of Zn(0) in ultrapure water. Other reducing agents that can be used according to the present invention are nitrate reductase; reducing agents in acid medium such as, for example, formic acid, Fe (0), and ammonium ions; and reducing agents in basic medium such as, for example, solutions of Al, Zn and Fe (II) and hydrazine, among many others.

In another aspect additional, the invention relates to a method for producing the ion gel of the invention comprising the following steps:
A)- preparing a mixture of the precursor monomers of the hydrogel and, where appropriate, a polymerisation initiator in an ionic liquid;
B)-polymerising the monomers in the ionic liquid to obtain the hydrogel in the ionic liquid, and
C)-incorporating the reactive system that provides a change in colour upon reaction with the nitrite into the hydrogel in the ionic liquid.

Depending on the monomers selected in each particular embodiment for polymerisation, one skilled in the art can select, where appropriate, a suitable initiator, as well as the polymerisation conditions such as UV or visible light, and/or temperature and the particular ionic liquid. As explained above, at least one monomer also acts as a cross-linking agent in the reaction. In a particular embodiment, the monomers are N-isopropylacrylamide and N,N'-methylenebis(acrylamide), and they are photopolymerised with a photopolymerisation initiator, such as 2,2-dimethoxy-2-phenylacetophenone and UV light. The polymerisation of step B) is carried out in an ionic liquid as defined above and the result is a hydrogel in said ionic liquid which, as explained below, serves as a "target" in the reservoir 3 of the device.

In a preferred embodiment, said ionic liquid is selected from 1-ethyl-3-methylimidazolium ethyl sulphate and trihexyltetradecyl phosphonium dicyanamide. On occasions it may be advisable or even necessary to heat the reaction mixture to facilitate dissolution of the monomers.

Once the hydrogel in the ionic liquid is obtained, it is optionally washed to remove the residues of unreacted monomers and of any other reagent with ultrapure water and/or a suitable organic solvent, for example an alcohol.

Then in step C), the reactive system is incorporated into the hydrogel in the ionic liquid, for example by simple arrangement or impregnation thereof. In a particular embodiment, the reactive system is the Griess reagent. The resulting ion gel is then left to dry. In a particular embodiment, the same amount in microlitres of Griess reagent as of the ion gel mixture is added thereto.

In another additional aspect, the invention relates to a method for producing the microfluidic device of the invention. The method comprises the steps of:
- providing on a substrate 1:
- one or more calibration reservoirs 2 that are separated from one another;
- a reservoir 6 for holding a sample to be analysed,
- a calibration reservoir 3 ("target"),
- a measuring reservoir 4,
- a measuring reservoir 7 for holding the sample to be analysed,
- placing a microfluidic channel 5 including a reducing agent between reservoir 6 and reservoir 4, and
- obtaining the ion gel *in situ* in the reservoirs 2, 4, and 7, and the hydrogel in the ionic liquid in the reservoir 3, according to the production method defined above.

A laser can be used in a conventional manner for producing the microfluidic device. Furthermore, there is arranged a microfluidic channel 5 which connects the reservoirs 6 and 4. Said channel consists of a particular embodiment of a microfluidic paper which allows the passage therethrough by capillarity of the sample which is held in the reservoir 6 towards the measuring reservoir 4. Said microfluidic channel 5 comprises a reducing agent capable of reducing the nitrate of the sample to nitrite, which species is the one detected and determined in the present invention. In a particular embodiment, said reducing agent is Zn(0) and can be incorporated into the channel 5 in the form of an emulsion in ultrapure water. The amount of emulsion which is incorporated can vary in each case particular; 1 to 20 µL, in particular 5 to 10 µL of suspension of reducing agent are typically incorporated into the channel 5.

The hydrogel in the ionic liquid is obtained directly *in situ* in each reservoir 2, 3, 4, 6 and 7 of the microfluidic device, following the method for producing the ion gel of the invention described above (steps A and B). The ion gel is obtained in the reservoirs 2, 4, 6 and 7 by incorporating the reactive system (according to step C).

In this sense, to obtain the hydrogel in the ionic liquid, first a mixture of the precursor monomers, and, where appropriate, a polymerisation initiator, in an ionic liquid is obtained. The mixture can be obtained *in situ* in each reservoir or can be obtained in a separate vessel and an amount of mixture which is held in the corresponding reservoirs can be taken from it. Then polymerisation is carried out *in situ* in the device. Once the polymerisation reaction has ended, the resulting product is washed; then the reactive system is incorporated into reservoirs 2, 4, 6 and 7 (but not reservoir 3) and left to dry. The amount of mixture that is held in the corresponding reservoirs is variable and depends in each case on the design of the microfluidic device. The volumes are typically between 1 µL and 20 µL of mixture, for example between 5 and 10 µL.

The method for producing the microfluidic device of the invention can further comprise an additional step of incorporating into the ion gel in the calibration reservoirs 2 calibration solutions with different concentrations of nitrite (standard solutions), in which a change in colour is caused upon reaction of the reactive system with the nitrite that is proportional in colour intensity to the concentration of each solution in a linear range. A calibration curve is obtained from processing the changes in colour in the calibration reservoirs 2 as explained below and is used to colorimetrically determine the concentration of nitrite in the analysed sample. Figure 3 shows a particular embodiment where calibration solutions with different concentrations of nitrite were used, such that 0 ppm were arranged in a first reservoir 2, 2.5 ppm were arranged in the second reservoir 2, 5 ppm were arranged in the third reservoir 2, 7.5 ppm were arranged in the fourth reservoir 2 and 10 ppm of nitrite were arranged in the fifth.

In another aspect, the invention relates to a method for colorimetrically determining and detecting the concentration of nitrite and/or nitrate in a sample, hereinafter method of the present invention. The method of the invention comprises the use of the microfluidic device of the invention, allows colorimetrically determining and detecting the concentration of nitrite in a sample and/or the concentration of nitrate in the sample.

The method is defined below in reference to the microfluidic device produced as described above.

The method of the invention comprises the following steps:
(i)- holding equal amounts of a sample to be analysed in the reservoirs 3, 6 and 7 of the microfluidic device of the invention,
(ii)- reducing the nitrate present in the sample held in the reservoir 6 to nitrite upon the action of the reducing agent as it passes through the microfluidic channel 5;
(iii)- colorimetrically determining the concentrations of nitrite in the calibration reservoirs 2 and establishing the calibration curve;
(iv)- colorimetrically determining and detecting the concentration of nitrite present in the sample in the reservoir 7 and the concentration of nitrite in the sample in the reservoir 4, and
(v)- determining the concentration of nitrate in the sample by the difference between the concentration obtained in the reservoir 7 and the concentration obtained in the reservoir 4.

A particular embodiment is based on the microfluidic device in which the calibration solutions having different concentrations of nitrite have already been incorporated in the calibration reservoirs 2, and therefore this step would not be a step of the method of the invention. Another particular embodiment is based on the microfluidic device in which the calibration solutions with different concentrations of nitrite have not yet been incorporated in the calibration reservoirs 2, and therefore this step is defined and understood as an additional step of the method of the invention.

The colorimetric detection of nitrite and/or nitrate in the sample can be done with the naked eye if the reservoirs 4 and 7 develop a colour. In a particular embodiment, the volume of a sample to be analysed which is arranged in the reservoirs 3, 6 and 7 is the same.

The determination of concentrations is done by analysing the colour of the reservoirs 2, 4 and 7 from an image taken by means of a camera or video of the microfluidic device 1, and processing the different reservoirs of the device to determine the concentrations. In a particular embodiment of the determination method, the invention takes into account the value determined in the reservoir 3 ("target").

The sample that is analysed is liquid and it can be any taken from any liquid of a natural, industrial or municipal source which may have these nitrite/nitrate contaminants.

In another aspect, the invention relates to the use of the ion gel of the invention and/or to the use of the microfluidic device of the invention for colorimetrically determining and detecting the concentration of nitrite and/or nitrate in a sample.

The use of the ion gel and of the device allows carrying out in a single unit the arrangement (for example by injection) of the sample to be analysed, the chemical reactions and the detection and determination of the analyte in a single step. The device is produced from a substrate of a low-cost flexible material and can be easily modified depending on the desired structure. The use thereof also has other advantages, including its easy storage, transport and disposability, which is extremely suitable for the quick and inexpensive *in situ* diagnosis by untrained personnel personal, without requiring an energy source or electronic components, and which can be easily interrogated with a photographic camera. Furthermore, as illustrated below in the Examples, the use thereof provides high sensitivity and reliability.

The device has very small dimensions, is portable, and uses small sample volumes, thereby reducing the amount of reagents and providing a response in a short period of time.

The calibration points (number of reservoirs 2) are variable and are included in the device itself. Handling is simple and requires minimal manipulation for the *in situ* characterisation of the analyte to be analysed from actual samples (for example, contaminated water).

The range of application can vary and be selected in each case. Generally typical ranges of water contaminated by nitrates which range from 1 ppb to several ppm are determined.

Illustrative examples of the invention are provided below which must not be interpreted in any case as being limiting of the scope of protection of the invention.

### Examples

N-isopropylacrylamide, N,N'-methylene-bis(acrylamide) were used to produce ion gels, and 2,2-dimethoxy-2-phenylacetophenone was used as photoiniciator. The ionic liquids used were ethyl sulphate 1-ethyl-3-methylimidazolium and trihexyltetradecyl-phosphonium dicyanamide (Sigma-Aldrich, Spain).

Griess reagent. The Griess reagent is commercially available, but it can be prepared in a conventional manner by mixing sulphanilamide, naphthylenediamine dihydrochloride, and phosphoric acid.

The UV light source used for photopolymerisation was BONDwand UV-365 nm (Electrolyte Corporation, USA).

The UV-Vis spectra were recorded in a 900 UV-VIS-NIR Perkin-Elmer Lambda spectrometer.

The photos were taken with a Canon EOS 1000D camera and calibrated by means of using an X-Rite card (X-Rite Inc., USA.) with Color Checker Passport v. 1.0.2 programme (X-Rite Inc., USA.) and followed by Photoshop CC programme (Adobe Photoshop CS5 Extended, Adobe Systems Inc., USA.).

The spectra of ion gels and with nitrite were taken for the characterisation of storage of the Griess reagent in ion gel. It was found that the maximum absorbance for the Griess reaction was at λ = 532 nm. Figures 2A and 2B show the difference between the absorbance of the ion gel and the ion gel with nitrite.

### Example 1: Producing the device (see Figure 1).

The sensor was produced from a 1 mm thick poly(methyl)methacrylate (PMMA) plate (Goodfellow, United Kingdom) which was cut with a CO2 laser ablation system (Universal Laser Systems, Austria), establishing both the size of the device (rectangle) and the different components of said device (reservoirs) using different laser energies.

Each device was designed with five calibration reservoirs 2, a reservoir 3 for calibrating the hydrogel in the ionic liquid in contact with the sample ("target"), a reservoir 6 for holding the sample to be analysed; a measuring reservoir 4, and a measuring reservoir 7 for measuring and for also holding the sample to be analysed. The device was 1 mm thick and had a size of 22 mm x 10 mm.

A microfluidic channel 5 which connected the reservoirs 6 and 4 to one another was produced from grade 595 Whatman filter paper and a metallic Zn emulsion (Sigma-Aldrich, Spain) in ultrapure water was arranged in same.

### Preparation of the hydrogel and ion gel

Two hydrogels in an ionic liquid were synthesised by means of the mixture of N-isopropylacrylamide, N,N'-methylene-bis(acrylamide) and a photoiniciator (2,2-dimethoxy-2-phenylacetophenone) dissolved in the ionic liquids (IL): 1-ethyl-3-methylimidazolium sulphate acetate or trihexyltetradecyl phosphonium dicyanamide respectively, by means of heating at 45°C for 10 min.

5 µl of the preceding mixtures were held in the reservoirs of the device and drop casting is carried out by photopolymerisation *in situ* (1600 mW cm-2) for 20 and 30 minutes, respectively.

The hydrogels in IL were washed thoroughly with ultrapure water and ethanol, and all except the reservoir 3 were embedded with Griess reagent and left to dry for 12 hours, obtaining two ion gels (IO-1 and IO-2).

3 µL of standard solutions of nitrite were added to the ion gels of the calibration reservoirs 2, said solutions being prepared with the following concentrations in the linear range of 0-10 ppm: (see Figure 3): (0 ppm), (2.5 ppm), (5 ppm), (7.5 ppm) and (10 ppm).

After a few minutes, different colour intensities developed in each calibration reservoir 2, proportional in intensity to each of the concentrations of nitrite. The same amount in microlitres of liquid sample to be analysed was held in reservoirs 3 and 6 and 7 with an amount of nitrate of 2.5 ppms and of nitrite of 2.5 ppms.

The sample held in 6 was moved by capillarity to reservoir 4 via the microfluidic channel 5 where Zn(0) reduced all the nitrate present in the sample upon its passage to nitrite.

Given colour intensities developed in reservoirs 4 and 7 on average.

Then photos of the device were taken with the camera and were processed by imaging analysis.

The parameters of luminance (L), chromaticity (C) and hue (H) were taken by means of PhotoShop CC by pixelling each reservoir and the concentration of nitrate of the sample was calculated using a multivariate calibration model (see Figure 3).

In particular, the concentration of nitrite (determined in the reservoir 7), the concentration of nitrite plus nitrate reduced to nitrite (determined in the reservoir 4) were determined, and the concentration of nitrate was determined from the difference. The effect of the sample on the hydrogel in the ionic liquid ("target") in the reservoir 3 was also evaluated.

The obtained results showed a concentration of 5 ± 0.5 ppm of nitrite (in the reservoir 4) which is in accordance with the concentration of the sample of nitrite plus that of nitrate, added to the reservoir 6.

The concentration of nitrite determined in the reservoir 7 was 2.5 ± 0.5 ppm.

Therefore, the difference between both (reservoir 4 minus reservoir 7) resulted in a concentration of nitrate in the sample of 2.5 ± 0.6 ppm.

The invention is not limited to the specific embodiments which have been described, but it also covers, for example, variants which can be performed by one skilled in the art based on which is deduced from the claims.

## Claims

1. An ion gel comprising:
- a hydrogel in an ionic liquid, and
- a reactive system providing a change in colour upon reaction with the nitrite.

2. The ion gel according to claim 1, wherein the hydrogel is a polyacrylamide.

3. The ion gel wherein the hydrogel is obtained by polymerisation of the monomers N-isopropylacrylamide and N,N'-methylene-bis(acrylamide).

4. The ion gel according to one of claims 1 to 3, wherein the reactive system is the Griess reagent.

5. The ion gel according to any of claims 1 to 4, wherein the ionic liquid comprises a cation and an anion where the at least one cation is selected from: trihexyltetradecyl phosphonium, tributyl tetradecyl phosphonium, tetrabutyl phosphonium, triisobutyl methyl phosphonium, 1-butyl-1-methyl pyrrolidine, 1-ethyl-3-methylimidazolium, 1-butyl-3-methylimidazolium, N-propyl N-methyl-pyrrolidinium, N-butyl N-methyl-pyrrolidinium, trioctyl methyl ammonium, and the at least one anion is selected from tosylate, dodecylbenzenesulphonate, ethyl sulphate, bis(trifluoromethanesulphonyl) amide, dicyanamide, tetracyanoborate, hexafluorophosphate, tetrafluoroborate, ethyl sulphate, tris(pentafluorethyl)trifluorophosphate, chloride, bromide, iodide, fluoride.

6. The ion gel according to claim 5, wherein the ionic liquid is selected from 1-ethyl-3-methylimidazolium ethyl sulphate and trihexyltetradecyl phosphonium dicyanamide.

7. A microfluidic device comprising
a)- a substrate (1) having:
b)- two or more calibration reservoirs (2) that are separated from one another and include an ion gel,
c)- a calibration reservoir (3) comprising hydrogel in the ionic liquid,
d)- a reservoir (6) for holding a sample to be analysed, connected to a measuring reservoir (4) including an ion gel via a microfluidic channel (5) including a reducing agent,
e)- a measuring reservoir (7) including an ion gel where a sample to be analysed is held,
wherein the ion gel and the hydrogel in the ionic liquid is as it is defined in the preceding claims.

8. The microfluidic device according to claim 7, wherein the calibration reservoirs (2) that are separated from one another are at least five in number.

9. The microfluidic device according to claim 7 or 8, wherein the calibration reservoirs (2) contains different concentrations of nitrite, and wherein a change in colour is caused upon reaction of the reactive system with the nitrite that is proportional in colour intensity to the concentration of nitrite in a linear range.

10. The microfluidic device according to any one of claims 7 to 9, wherein the substrate (1) is a poly(methyl methacrylate) plate.

11. The microfluidic device according to any one of claims 7 to 10, wherein the microfluidic channel (5) comprises a microfluidic paper.

12. The microfluidic device according to any one of claims 7 to 11, wherein a reducing agent is arranged in the microfluidic channel (5).

13. The microfluidic device according to claim 12, wherein the reducing agent is Zn(0).

14. A method for producing the ion gel according to any one of claims 1 to 6, which method comprises the following steps:
A)- preparing a mixture of the precursor monomers of a hydrogel and, where appropriate, a polymerisation initiator in an ionic liquid;
B)-polymerising the monomers in the ionic liquid to obtain the hydrogel in the ionic liquid, and
C)-incorporating the reactive system that provides a change in colour upon reaction with the nitrite into the hydrogel in the ionic liquid.

15. The method for producing the ion gel according to the preceding claim, wherein the monomers are N-isopropylacrylamide and N,N'-methylene-bis(acrylamide), and they are photopolymerised with 2,2-dimethoxy-2-phenylacetophenone and UV light.

16. The method for producing the ion gel according to claim 14 or 15, wherein the ionic liquid is selected from 1-ethyl-3-methylimidazolium ethyl sulphate and trihexyltetradecyl phosphonium dicyanamide.

17. The method for producing the microfluidic device according to any one of claims 7 to 13, which method comprises:
- providing on a substrate (1):
- one or more calibration reservoirs (2) that are separated from one another;
- a reservoir (6) for holding a sample to be analysed,
- a calibration reservoir (3) for calibrating the hydrogel in the ionic liquid,
- a measuring reservoir (4),
- a measuring reservoir (7) for holding the sample to be analysed,
- placing a microfluidic channel (5) including a reducing agent between the reservoir (6) and the reservoir (4), and
- obtaining the hydrogel in the liquid *in situ* in reservoirs (2), (3), (4) and (7) and then the ion gel in reservoirs (2), (4) and (7) upon addition of the reactive system according to the method defined in claims 14 to 16.

18. The method according to claim 17, which method comprises the additional step of incorporating into the ion gel in the calibration reservoirs (2) calibration solutions having different concentrations of nitrite, wherein a change in colour is caused upon reaction of the reactive system with the nitrite that is proportional in colour intensity to the concentration in a linear range.

19. A method for colorimetrically determining and detecting the concentration of nitrite and/or nitrate in a sample, which method comprises the steps of:
(i)- holding equal amounts of a sample to be analysed in the reservoirs (3), (6) and (7) of the microfluidic device of the invention,
(ii)- reducing the nitrate present in the sample held in the reservoir (6) to nitrite upon the action of the reducing agent as it passes through the microfluidic channel (5);
(iii)- colorimetrically determining the concentrations of nitrite in the calibration reservoirs (2) and establishing the calibration curve;
(iv)- colorimetrically determining and detecting the concentration of nitrite present in the sample in the reservoir (7) and the concentration of nitrite in the sample in the reservoir (4), and
(v)- determining the concentration of nitrate in the sample by the difference between the concentration obtained in the reservoir (7) and the concentration obtained in the reservoir (4).

20. The method according to claim 19, wherein the determination of the concentrations is performed by analysing the colour of the reservoirs (2), (4) and (7) from an image taken by means of a camera or video of the microfluidic device (1), and processing the different reservoirs of the device to determine the concentrations.

21. Use of the ion gel and the microfluidic device according to any one of claims 1 to 6 and 7 to 13, respectively, for colorimetrically determining and detecting the concentration of nitrite and/or nitrate in a sample.
